# EUROPEAN PATENT APPLICATION

(11) **EP 4 674 590 A1**
(43) Date of publication of application: **07.01.2026**
(21) Application number: 23925397.4
(22) Date of filing: 07.12.2023
(51) Int. Cl.: B29C 45/37, A61M 5/30, B29C 33/42

(54) **RESIN MOLDED PRODUCT HAVING THROUGH-HOLE, MOLDING METHOD THEREFOR, MOLD, AND NEEDLELESS SYRINGE USING RESIN MOLDED PRODUCT**

(30) Priority: 02.03.2023 JP 2023031610
(71) Applicant: Daicel Corporation, Osaka-shi, Osaka 530-0011 (JP)
(72) Inventor: FUKUI, Sadayuki, Tokyo (JP)
(74) Representative: Grünecker Patent- und Rechtsanwälte PartG mbB
(86) International application number: PCT/JP2023/043896
(87) International publication number: WO 2024/180845

(57) **Abstract**

A resin molded product is molded by injection molding. A through hole is open in a predetermined end surface of the resin molded product. A protruding portion protruding from the predetermined end surface in an axial direction of the through hole is formed in an annular shape along an opening edge of the through hole.

## Description

### Technical Field

The present invention relates to a resin molded product having a through hole, a molding method for the resin molded product, a mold, and a needleless injector using the resin molded product.

### Background Art

In the related art, as a molding method for a resin molded product having a through hole, a method is proposed that uses injection molding in which a core pin having a through hole shape is inserted into a molten resin present in a cavity to mold the product (Patent Document 1, for example).

### Citation List

### Patent Document

Patent Document 1: JP 2017-109471 A
Patent Document 2: JP 2013-166363 A

### Summary of Invention

### Technical Problem

There is a concern that, when molding a resin molded product having a through hole by injection molding, minute burrs are generated at an opening portion of the through hole in inserting a core pin into a molten resin and forming the hole, and the minute burrs peel off when the molded product is used.

A technology of the present disclosure has been made in view of the above-described circumstances, and an object thereof is to provide a resin molded product having a through hole, in which generation of minute burrs at an opening portion of the through hole is suppressed.

### Solution to Problem

To solve the above problems, the technology of the present disclosure adopts the following configurations. That is, a resin molded product as one aspect of the technology according to the present disclosure is a resin molded product molded by injection molding. A through hole is open in a predetermined end surface of the resin molded product, and a protruding portion protruding from the predetermined end surface in an axial direction of the through hole is formed along an opening edge of the through hole.

In the resin molded product described above, in the protruding portion, a thickness of the protruding portion in a direction orthogonal to the axial direction may be from 1 µm to 1000 µm.

Further, in the resin molded product described above, in the protruding portion, a thickness of the protruding portion in a direction orthogonal to the axial direction may be from 3 µm to 100 µm.

Further, in the resin molded product described above, in the protruding portion, a thickness of the protruding portion in a direction orthogonal to the axial direction may be from 5 µm to 15 µm.

Further, the resin molded product described above may be formed as a container, in which an injection intended substance is stored, in a needleless injector configured to inject the injection intended substance into a target region without passing through an injection needle. The through hole may define a flow path of the injection intended substance, and the injection intended substance may be injected into the target region, using an opening portion of the through hole on the predetermined end surface as an ejection port.

The technology according to the present disclosure may be a molding method for a resin molded product in which a resin molded product is molded by injection molding, the resin molded product having a through hole formed therein, the through hole being open in a predetermined end surface. The molding method includes:
closing a mold; and injecting a resin into the mold. The mold includes a cavity having a space formed therein in which the resin is to be injected, and a core inserted into the space by moving relative to the cavity in a predetermined first direction, and defining a region, into which the resin does not flow, inside the space. The core includes a core pin extending in the first direction of the mold, and defining a shape of the through hole inside the space. The cavity includes an end-surface defining surface facing the space and defining a shape of the predetermined end surface of the resin molded product. A core-pin receiving hole is formed in the end-surface defining surface, the core-pin receiving hole including a recessed portion and a receiving portion, the recessed portion being open to the space, being recessed in the first direction, and having an inner wall portion formed in the recessed portion, the receiving portion being open to the recessed portion, extending in the first direction, and being configured to receive a distal end of the core pin of the core inserted into the space. An opening width of the recessed portion is set to be larger than an opening width of the receiving portion. In the closing of the mold, the core is inserted into the space of the cavity by the core moving relative to the cavity in the first direction, and in the injecting of the resin, a protruding portion protruding from the predetermined end surface of the resin molded product is formed along an opening edge of the through hole by the recessed portion receiving the resin injected into the space in such a manner that the resin does not reach the receiving portion.

In the molding method for a resin molded product described above, in a state where the core pin is inserted into the core-pin receiving hole, a distance between the core pin and an inner wall surface of the recessed portion at an opening portion of the recessed portion may be from 10 µm to 100 µm.

In the molding method for a resin molded product described above, in a state where the core pin is inserted into the core-pin receiving hole, a distance between the core pin and an inner wall surface of the recessed portion at an opening portion of the recessed portion may be from 5 µm to 15 µm.

In the molding method for a resin molded product described above, in a state where the core pin is inserted into the core-pin receiving hole, a distance between the core pin and an inner wall surface of the receiving portion at an opening portion of the receiving portion may be 10 µm or less.

In the molding method for a resin molded product described above, in a state where the core pin is inserted into the core-pin receiving hole, a distance between the core pin and an inner wall surface of the receiving portion at an opening portion of the receiving portion may be 5 µm or less.

In the molding method for a resin molded product described above, in a state where the core pin is inserted into the core-pin receiving hole, a distance between the core pin and an inner wall surface of the receiving portion at an opening portion of the receiving portion may be 2.5 µm or less.

In the molding method for a resin molded product described above, a depth of the recessed portion from the end-surface defining surface in the axial direction may be 20 µm or more.

In the molding method for a resin molded product described above, a depth of the recessed portion from the end-surface defining surface in the axial direction may be 50 µm or more.

In the molding method for a resin molded product described above, a depth of the recessed portion from the end-surface defining surface in the axial direction may be 100 µm or more.

In the molding method for a resin molded product described above, a shape of an opening portion of the through hole and a shape of the recessed portion in a cross section orthogonal to the first direction may be similar to each other. In the closing of the mold, in a state where the core pin is inserted into the core-pin receiving hole, the inner wall portion of the recessed portion may face and extend along a lateral surface of the core pin.

In the molding method for a resin molded product described above, an opening portion of the recessed portion may have an elliptical shape in a cross section orthogonal to the first direction.

In the molding method for a resin molded product described above, in the first direction, the recessed portion may be formed in a columnar shape extending in a direction in which the core pin is inserted into the core-pin receiving hole.

The technology according to the present disclosure may be a mold for a resin molded product, the mold being used for molding a resin molded product by injection molding, the resin molded product having a through hole formed therein, the through hole being open in a predetermined end surface. The mold includes: a cavity having a space formed therein in which resin is to be injected, and a core inserted into the space by moving relative to the cavity in a predetermined first direction, and defining a region, into which the resin does not flow, inside the space. The core includes a core pin extending in the first direction and defining a shape of the through hole inside the space. The cavity includes an end-surface defining surface facing the space and defining a shape of the predetermined end surface of the resin molded product. A core-pin receiving hole is formed in the end-surface defining surface, the core-pin receiving hole including a recessed portion and a receiving portion, the recessed portion being open to the space and being recessed in the first direction, the receiving portion being open to the recessed portion, extending in the first direction, and being configured to receive a distal end of the core pin of the core inserted into the space. An opening width of the recessed portion is set to be larger than an opening width of the receiving portion, and a protruding portion protruding from the predetermined end surface of the resin molded product is formed along an opening edge of the through hole by the recessed portion receiving the resin injected into the space in such a manner that the resin does not reach the receiving portion.

The technology according to the present disclosure may be a needleless injector including the resin molded product described above, the resin molded product being formed as a container in which an injection intended substance is stored. The needleless injector is configured to inject the injection intended substance into a target region without passing through an injection needle.

### Advantageous Effects of Invention

According to the present disclosure, it is possible to provide a resin molded product in which minute burrs are less likely to be generated at an opening portion of a through hole.

### Brief Description of Drawings

FIG. 1 is a schematic cross-sectional view of a resin molded product according to an embodiment.
FIG. 2 is a schematic cross-sectional view of a mold and a resin molded product according to the embodiment.
FIG. 3 is a partially enlarged view of FIG. 2.
FIG. 4 is a partially enlarged view of FIG. 2.
FIG. 5 is a flowchart of a molding method for a resin molded product according to the embodiment.
FIG. 6 is a schematic cross-sectional view of a needleless injector using the resin molded product according to the embodiment.

### Description of Embodiments

An embodiment of the present disclosure is described below with reference to the drawings. Note that respective configurations and combinations thereof in the present embodiment are merely examples, and additions, omissions, substitutions, and other changes to the configurations can be made as appropriate without departing from the gist of the present invention. Further, the present disclosure is not limited by the embodiment, and is limited only by the claims. In the embodiment described below, a resin molded product according to the present disclosure is applied to a container of a needleless injector, in which an injection intended substance is stored, but the technology of the present disclosure is not limited thereto. That is, the technology according to the present disclosure is not limited to the container of the needleless injector, and can be applied to a resin molded product having a through hole.

### Device Configuration

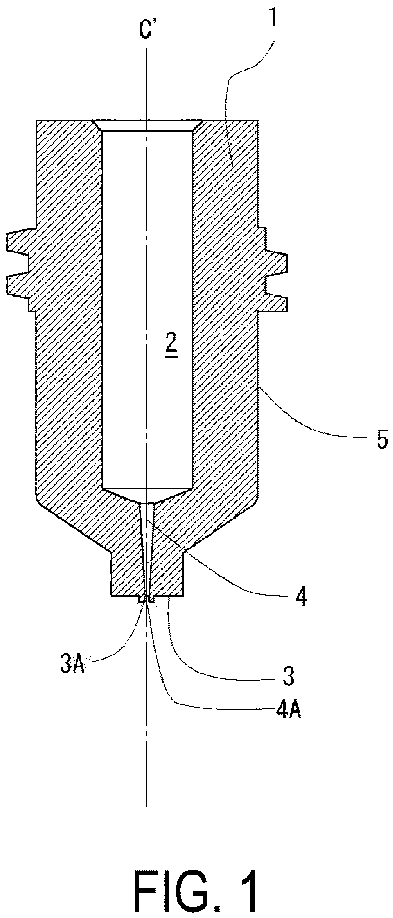

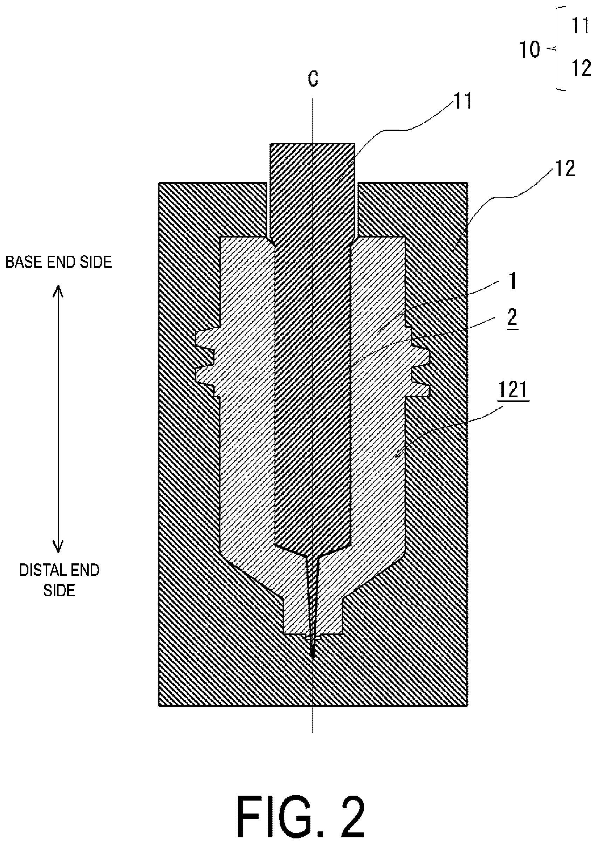

FIG. 1 is a schematic cross-sectional view of a resin molded product 1 according to the embodiment. FIG. 2 is a cross-sectional view of a mold according to the embodiment, taken along a center axis C. FIG. 2 illustrates an example when the resin molded product 1 illustrated in FIG. 1 is molded by a mold 10 used at the time of injection molding. In the present embodiment, a direction in which a core 11 is inserted relative to a cavity 12 in closing a mold to be described later corresponds to a "predetermined first direction". In the present embodiment, the "first direction" is a direction parallel to the center axis C of the mold 10 and a center axis C' of a through hole of the resin molded product 1. The mold 10 can mold the resin molded product 1 through injecting a molten resin into an internal space 121 subsequent to the closing of the mold. Further, for convenience of description of the embodiment, a direction in which the internal space 121 is open to receive the core 11 in the cavity 12 is referred to as a base end side, and a direction in which a core-pin receiving hole 124 is disposed in the cavity 12 is referred to as a distal end side. The base end side and the distal end side merely indicate a relative positional relationship between respective elements in the resin molded product 1 and the mold 10.

### Resin Molded Product

FIG. 1 is a schematic cross-sectional view of the resin molded product 1 according to the embodiment. The resin molded product 1 is a molded product obtained by molding a molten resin using the mold 10 to be described later. In the present embodiment, the resin molded product 1 is formed as a container for storing the injection intended substance used in the needleless injector. The center axis of a through hole 4 of the resin molded product 1 is referred to as the center axis C' for convenience. The resin molded product 1 is formed in a substantially cylindrical shape extending in the first direction. The resin molded product 1 includes a storage space 2 for storing a chosen substance, a predetermined end surface 3 that is a part defining the shape of the resin molded product 1, the through hole 4 which is open in the predetermined end surface 3, and a lateral surface portion 5 that is a part defining the shape of the resin molded product 1. The storage space 2 is formed as a hollow portion of the resin molded product 1. The predetermined end surface 3 constitutes a distal end surface of the resin molded product 1. The lateral surface portion 5 constitutes a lateral surface of the resin molded product 1. The through hole 4 is a through hole through which the storage space 2 communicates with an external space of the resin molded product 1, and is open in the predetermined end surface 3. More specifically, the through hole 4 is formed to extend toward the predetermined end surface 3, which is the distal end side of the storage space 2, along the center axis C' of the through hole 4, and an opening portion 4A is formed on the distal end side of the through hole 4. Further, in the present embodiment, the through hole 4 defines a flow path of the injection intended substance to be described later in a needleless injector 100 to be described later. The opening portion 4A of the through hole 4 is formed in a circular shape and used as an ejection port for the injection intended substance.

A protruding portion 3A protruding from the predetermined end surface 3 in the direction of the center axis C' is formed around the opening portion 4A. In the present embodiment, the protruding portion 3A is formed in an annular shape along an opening edge of the opening portion 4A. However, a modification may be adopted, for example, in which the opening portion 4A is formed in a circular shape, while the protruding portion 3A is formed in an elliptical shape or a polygonal shape. That is, in a direction orthogonal to the center axis C', the cross-sectional shape of the opening portion 4A (the cross-sectional shape of the inside of the opening) and the cross-sectional shape of the protruding portion 3A (the cross-sectional shape of a radially outer side edge portion of the protruding portion formed at the opening edge) may be different from each other. Further, the protruding portion 3A need not be annular, and may be a shape obtained by cutting out a part of the annular shape, such as a shape like a Landolt ring, for example, or may be formed intermittently. However, the shape of the opening portion 4A may be a polygonal shape or the like, and in this case also, the protruding portion 3A is preferably formed along the opening edge of the opening portion 4A.

By setting a distance t1 to be described later, a thickness of the protruding portion 3A in the direction orthogonal to the center axis C' is in a range from 1 µm to 1000 µm, is preferably in a range from 3 µm to 100 µm, and is even more preferably in a range from 5 µm to 15 µm. A rigid shape of a constant size as described above is formed as the protruding portion 3A along the opening portion 4A. This makes it possible to prevent generation of minute burrs.

### Mold

FIG. 2 is a cross-sectional view of the mold 10 according to the embodiment, taken along the center axis C. The mold 10 is configured to extend in the first direction. The mold 10 includes the cavity 12 having the internal space 121 for storing the molten resin, and includes the core 11 defining a region where the molten resin does not flow into the internal space 121. The core 11 is inserted into the cavity 12 by moving relative to the cavity 12 in the first direction in the closing of the mold space 121, so that a region, into which the molten resin does not flow in the injecting of the molten resin, is formed as the storage space 2 of the resin molded product 1.

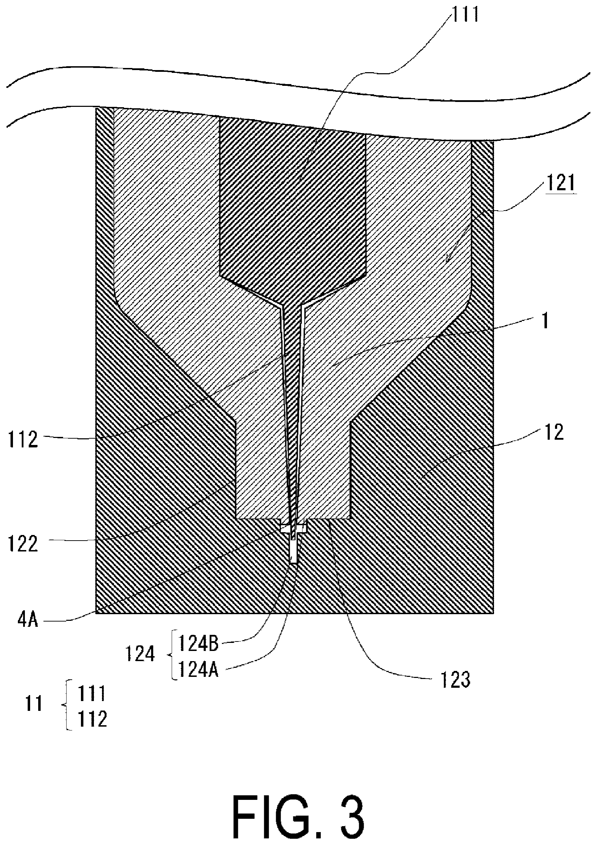

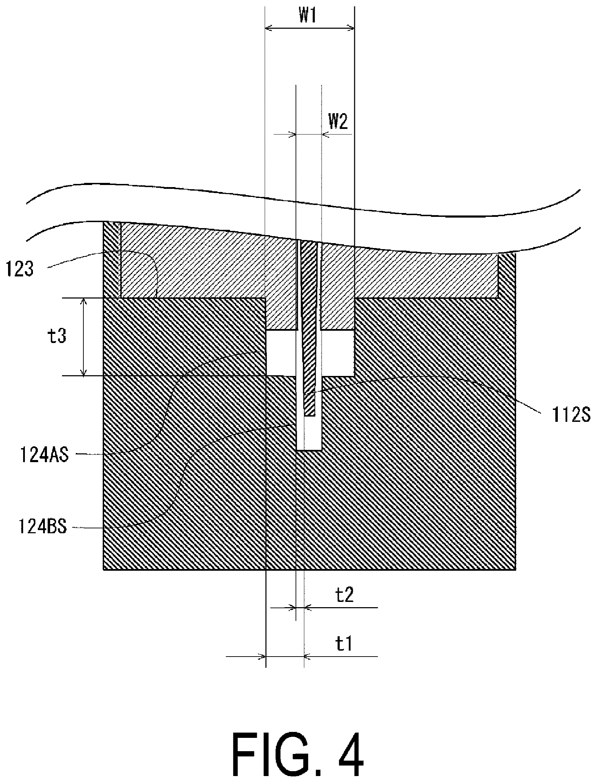

FIGS. 3 and 4 are partially enlarged views of FIG. 2. As illustrated in FIG. 3, the core 11 includes a core body 111 and a core pin 112, and extends in the first direction. The core body 111 forms the storage space 2 of the resin molded product 1 by occupying a part of the internal space 121 of the cavity 12 in the closing of the mold to be described later. In the present embodiment, the core pin 112 has a conical shape converging toward the distal end side, extends from the distal end side of the core main body 111, and defines the shape of the through hole 4 of the resin molded product 1. However, the shape of the core pin 112 is not limited, and may be, for example, a polygonal prism shape or an elliptical cylindrical shape.

The cavity 12 includes the internal space 121 for storing the molten resin, a lateral-surface defining surface 122 and an end-surface defining surface 123 both defining the internal space 121, and the core-pin receiving hole 124 for receiving the core pin 112. In the cavity 12, the shape of the lateral surface portion 5 of the resin molded product 1 is defined by the lateral-surface defining surface 122, and the shape of the predetermined end surface 3 is defined by the end-surface defining surface 123. Therefore, a portion on the distal end side from the end-surface defining surface 123 may be formed of a separate member to facilitate mold opening, thus facilitating removal of the resin molded product 1 molded by the mold 10. Further, the core-pin receiving hole 124 extends from the end-surface defining surface 123 toward the distal end side in the first direction and is open to the internal space 121. The core-pin receiving hole 124 includes a recessed portion 124A formed in a circular columnar shape and a receiving portion 124B. However, the shapes of the core-pin receiving hole 124 and the core pin 112 are not limited, and the shape of the recessed portion 124A is not limited to the circular columnar shape, and may be another columnar shape. For example, when the core pin 112 is formed in a polygonal shape as a prism-shaped core pin, the core-pin receiving hole 124 may be formed in a polygonal columnar shape having a similar cross-sectional shape in a cross section orthogonal to the first direction, or when the shape of the core pin 112 is the elliptical columnar shape, the core-pin receiving hole 124 may be formed in an elliptical columnar shape having a similar cross-sectional shape in a cross section orthogonal to the first direction. In any of these cases, the protruding portion 3A of the resin molded product 1 is preferably formed to conform to the shape of the opening portion 4A.

As illustrated in FIG. 4, the core-pin receiving hole 124 has a stepped structure. The core-pin receiving hole 124 includes the recessed portion 124A formed by an inner wall surface 124AS as a result of being open to the internal space 121 and being recessed in the first direction, and includes the receiving portion 124B that is open to the recessed portion 124A, extends in the first direction, and is formed by an inner wall surface 124BS. In the present embodiment, a lateral surface 112S of the core pin faces and extends along the inner wall surface 124AS, and has a shape forming a circular hole in the resin molded product 1. An opening width W1, which is an opening diameter of the recessed portion 124A, is formed to be larger than an opening width W2, which is an opening diameter of the receiving portion 124B. When a molten resin is injected into the internal space 121 in the injecting of the molten resin to be described later, a flow of the molten resin into the receiving portion 124B located further on the distal end side relative to the recessed portion 124A is reduced. The receiving portion 124B has a shape that receives the distal end of the core pin 112. When the core 11 is inserted into the cavity 12 in the closing of the mold to be described later, the distal end of the core pin 112 reaches further toward the distal end side relative to the recessed portion 124A. That is, the protruding portion 3A is formed by the molten resin accumulating in the recessed portion 124A, and the core pin 112 is positioned to penetrate the accumulated molten resin. As a result, the shape of the through hole 4 of the resin molded product 1 is defined by the core pin 112. At this time, the shape of the opening portion 4A of the through hole 4 of the resin molded product 1, which is formed by the core pin 112, is similar to the shape of the recessed portion 124A in the cross section orthogonal to the first direction. When the core 11 is inserted into the cavity 12, the inner wall surface 124AS of the recessed portion 124A faces and extends along the lateral surface 112S of the core pin 112. This can reduce a flow of the molten resin to the receiving portion 124B that is located further on the distal end side relative to the recessed portion 124A.

As described above, the opening width W1 of the recessed portion 124A is formed to be larger than the opening width W2 of the receiving portion 124B. Accordingly, even when the core pin 112 is inserted into the core-pin receiving hole 124 in a decentered state, the receiving portion 124B functions as a guide for the core pin 112. The core pin 112 and the recessed portion 124A are not worn, and the core pin 112 and the receiving portion 124B are worn first. That is, in the recessed portion 124A, by providing a large gap between the inner wall surface 124AS of the recessed portion 124A and the lateral surface 112S of the core pin 112, and accumulating the molten resin, even when the core pin 112 or the core-pin receiving hole 124 is worn due to continuous use or the like, it is still possible to prevent the generation of minute burrs near the opening portion 4A of the resin molded product 1 to be molded.

As described above, the receiving portion 124B functions as a guide for the core pin 112. Thus, compared to a case in which the opening width W1 of the recessed portion 124A and the opening width W2 of the receiving portion 124B are formed to be the same opening width, and the core-pin receiving hole 124 does not have the stepped structure, it is possible to reduce breakage of the core pin 112 due to collision between the core pin 112 and the end-surface defining surface 123, or due to deformation resulting from an expansion of the mold 10 or the like caused by heat of the molten resin. Since the opening width of the recessed portion 124A is formed to be larger than the opening width of the receiving portion 124B, there is a margin for adjustment when inserting the core pin 112 into the core-pin receiving hole 124, which is useful in aiding the insertion. That is, the core pin 112 can be reliably inserted into the core-pin receiving hole 124, and thus the through hole 4 can be more reliably formed.

As illustrated in FIG. 4, in the present embodiment, in the core-pin receiving hole 124, a distance t1 between the lateral surface 112S of the core pin 112 and the inner wall surface 124AS of the recessed portion 124A in a state in which the core pin 112 has been inserted may be in a range from 10 µm to 100 µm, and more preferably in a range from 5 µm to 15 µm at the opening portion of the recessed portion 124A that is on the same plane as the end-surface defining surface 123. In this way, it is easy to form the protruding portion 3A of the resin molded product 1 described above such that the thickness in the direction orthogonal to the center axis C' is in the range from 1 µm to 1000 µm, preferably in the range from 3 µm to 100 µm, and more preferably in the range from 5 µm to 15 µm. It is thus possible to impart a rigid shape of a constant size to the protruding portion 3A of the resin molded product 1, thus preventing the generation of minute burrs.

In the present embodiment, in the core-pin receiving hole 124, a distance t2 between the lateral surface 112S of the core pin 112 and the inner wall surface 124BS of the receiving portion 124B in a state in which the core pin 112 has been inserted may be 10 µm or less, preferably 5 µm or less, and more preferably 2.5 µm or less. In this way, an inflow of the molten resin into the receiving portion 124B is reliably suppressed, and it is possible to prevent the generation of minute burrs near the opening portion 4A of the resin molded product 1.

In the present embodiment, in the recessed portion 124A of the core-pin receiving hole 124, a depth t3 from the end-surface defining surface 123 toward the distal end side may be 20 µm or more, preferably 50 µm or more, and more preferably 100 µm or more. In this way, it is possible to intentionally retain the molten resin in the recessed portion 124A, and reduce the molten resin reaching the receiving portion 124B, thus preventing the generation of minute burrs.

The shape of the opening portion of the recessed portion 124A of the core-pin receiving hole 124 may be an elliptical shape or the like in the cross section orthogonal to the first direction. The inner wall surface 124AS of the recessed portion 124A may be formed in a conical shape converging toward the distal end side, which is the closed end side of the core-pin receiving hole 124, or may be formed in a domed funnel-like shape bulging toward the side surface defining surface 122. As described above, as long as the configuration can impart a rigid shape of a constant height and size to the protruding portion 3A of the resin molded product 1, the shape may be modified depending on the application. In the present embodiment, since the recessed portion 124A is formed in a columnar shape, the cross section thereof taken along the first direction is rectangular. This can facilitate the manufacture of the mold. When the recessed portion 124A is formed in a conical shape converging toward the distal end side, this aids in guiding the core pin 112 when inserting the core pin 112 into the receiving portion 124B.

### Molding Method for Resin Molded Product

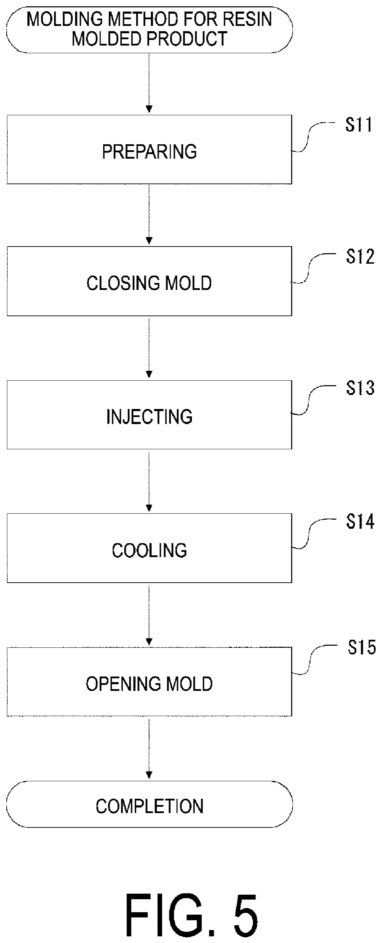

Next, a molding method for the resin molded product 1 according to the embodiment will be described. However, the molding method for the resin molded product 1 according to the present disclosure is not limited to the following method. FIG. 5 is a flowchart of the molding method for the resin molded product 1 according to the embodiment. Hereinafter, the description will be made with reference to FIG. 5.

First, in preparing in step S11, a molten resin that is the material of the resin molded product 1 is prepared. For example, a raw material such as resin pellets is melted to prepare for a subsequent step.

Next, in closing a mold in step S12, the core 11 is moved relative to the cavity 12 to be inserted into the cavity 12. When the mold 10 is closed, the core 11 is disposed to occupy a part of the internal space 121 of the cavity 12, thus defining a region into which the molten resin does not flow in a subsequent step. In this way, the region is formed as the storage space 2 and the through hole 4.

Next, in injecting in step S13, a nozzle (not illustrated) is pressed against a given molten-resin inlet (not illustrated) of the mold to pour the molten resin, thus forming the molten resin into the shape of the resin molded product 1.

Next, in cooling the molten resin in step S14, the resin is brought into a state of being defined in the shape of the resin molded product 1. By being subjected to the cooling of the molten resin, the shape of an object having been the molten resin with fluidity is fixed including the through hole 4, the protruding portion 3A, and the like, as the resin molded product 1.

Subsequently, in opening a mold in step S15, the core 11 is moved relative to the cavity 12 to be pulled out from the cavity 12, and the mold is opened. Thus, the resin molded product 1 is obtained.

### Needleless Injector

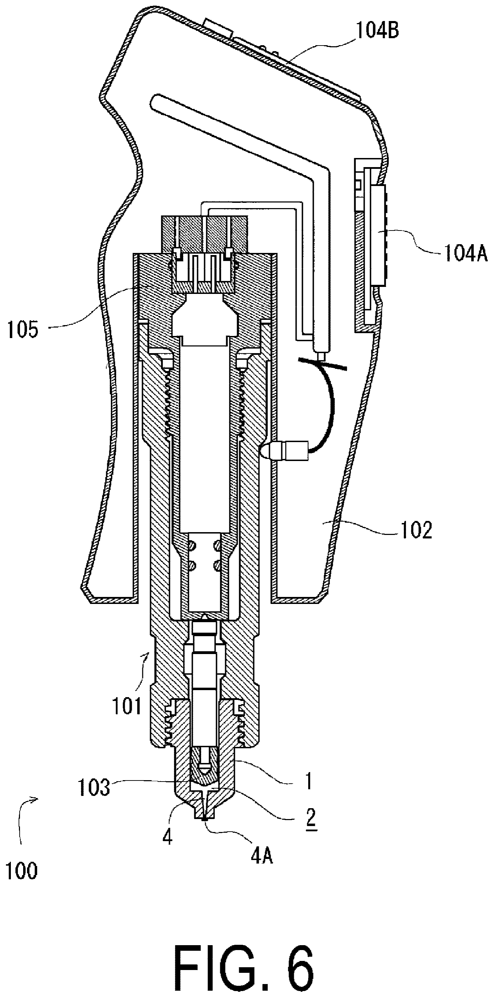

FIG. 6 is a schematic cross-sectional view of the needleless injector 100 using the resin molded product 1 according to the embodiment. In the present embodiment, the resin molded product 1 is formed as a container for storing the injection intended substance used in the needleless injector 100. FIG. 6 illustrates a cross-sectional view when the resin molded product 1 is fitted into the needleless injector 100.

Examples of a predetermined substance included in the injection intended substance include an organism-derived substance and a substance exhibiting a desired bioactivity that can be injected into a target region that is an organism. Examples of the organism-derived substance include DNA, RNA, nucleic acid, an antibody, and a cell. Examples of the substance exhibiting a desired bioactivity include substances exerting various types of pharmacological or therapeutic effects, such as medicines composed of low molecular compounds, proteins, and peptides, a vaccine, an inorganic substance such as metal particles for thermotherapy or radiotherapy, and a carrying body functioning as a carrier. Further, the liquid being the medium of the injection solution is only required to be a substance suitable for administering the predetermined substance exemplified by those substances to the target region, and may be aqueous or oleaginous, which is not limited. Furthermore, as long as the predetermined substance can be injected with the needleless injector 100, the viscosity of a liquid that is a medium is not particularly limited.

In the needleless injector 100, an injector assembly 101 is configured to be freely attachable to and detachable from a housing 102. The storage space 2 present between the container (the "resin molded product 1" in the present embodiment) included in the injector assembly 101 and a plunger 103 is filled with an injection solution during a preparation stage before the operation of the needleless injector 100. The injector assembly 101 is a unit that is replaced each time the injection solution is injected.

When the needleless injector 100 is operated, the above-described injection intended substance passes through the through hole 4, and is injected into the target region of an injection target, with the opening portion 4A used as an ejection port. That is, in the resin molded product 1, the through hole 4 is defined as a flow path for the injection intended substance, and the opening portion 4A functions as the ejection port for the injection intended substance. In the present embodiment, an ignition current is supplied to an igniter 105 based on operations of a first switch 104A and a second switch 104B, and the injection intended substance is injected into the target region without passing through an injection needle. By using the technique of the present disclosure, it is possible to prevent the minute burrs from becoming peeled off from the resin molded product 1 at the time of injecting the injection intended substance.

The embodiment of the present disclosure has been described above, and each of the aspects disclosed in the present specification can be combined with any other features disclosed therein.

### Reference Signs List

1 Resin molded product
2 Storage space
3 Predetermined end surface
4 Through hole
10 Mold
11 Core
112 Core pin
12 Cavity
124 Core-pin receiving hole
100 Needleless injector
C, C' Center axis

## Claims

1. A resin molded product molded by injection molding, wherein
a through hole is open in a predetermined end surface of the resin molded product, and
a protruding portion protruding from the predetermined end surface in an axial direction of the through hole is formed along an opening edge of the through hole.

2. The resin molded product according to claim 1, wherein
in the protruding portion, a thickness of the protruding portion in a direction orthogonal to the axial direction is from 1 µm to 1000 µm.

3. The resin molded product according to claim 1, wherein
in the protruding portion, a thickness of the protruding portion in a direction orthogonal to the axial direction is from 3 µm to 100 µm.

4. The resin molded product according to claim 1, wherein
in the protruding portion, a thickness of the protruding portion in a direction orthogonal to the axial direction is from 5 µm to 15 µm.

5. The resin molded product according to claim 1, wherein
the resin molded product is formed as a container, in which an injection intended substance is stored, in a needleless injector configured to inject the injection intended substance into a target region without passing through an injection needle,
the through hole defines a flow path of the injection intended substance, and the injection intended substance is injected into the target region, using an opening portion of the through hole on the predetermined end surface as an ejection port.

6. A molding method for a resin molded product in which a resin molded product is molded by injection molding, the resin molded product having a through hole formed therein, the through hole being open in a predetermined end surface, the molding method comprising:
closing a mold; and
injecting a resin into the mold, wherein
the mold includes
a cavity having a space formed therein in which the resin is to be injected, and
a core inserted into the space by moving relative to the cavity in a predetermined first direction, the core defining a region, into which the resin does not flow, inside the space,
the core includes a core pin extending in the first direction and defining a shape of the through hole inside the space,
the cavity includes an end-surface defining surface facing the space and defining a shape of the predetermined end surface of the resin molded product,
a core-pin receiving hole is formed in the end-surface defining surface, the core-pin receiving hole including a recessed portion and a receiving portion, the recessed portion being open to the space, being recessed in the first direction, and having an inner wall portion formed in the recessed portion, the receiving portion being open to the recessed portion, extending in the first direction, and being configured to receive a distal end of the core pin of the core inserted into the space,
an opening width of the recessed portion is set to be larger than an opening width of the receiving portion,
in the closing of the mold, the core is inserted into the space of the cavity by the core moving relative to the cavity in the first direction, and
in the injecting of the resin, a protruding portion protruding from the predetermined end surface of the resin molded product is formed along an opening edge of the through hole by the recessed portion receiving the resin injected into the space in such a manner that the resin does not reach the receiving portion.

7. The molding method for a resin molded product according to claim 6, wherein
in a state where the core pin is inserted into the core-pin receiving hole, a distance between the core pin and an inner wall surface of the recessed portion at an opening portion of the recessed portion is from 10 µm to 100 µm.

8. The molding method for a resin molded product according to claim 6, wherein
in a state where the core pin is inserted into the core-pin receiving hole, a distance between the core pin and an inner wall surface of the recessed portion at an opening portion of the recessed portion is from 5 µm to 15 µm.

9. The molding method for a resin molded product according to claim 6, wherein
in a state where the core pin is inserted into the core-pin receiving hole, a distance between the core pin and an inner wall surface of the receiving portion at an opening portion of the receiving portion is 10 µm or less.

10. The molding method for a resin molded product according to claim 6, wherein
in a state where the core pin is inserted into the core-pin receiving hole, a distance between the core pin and an inner wall surface of the receiving portion at an opening portion of the receiving portion is 5 µm or less.

11. The molding method for a resin molded product according to claim 6, wherein
in a state where the core pin is inserted into the core-pin receiving hole, a distance between the core pin and an inner wall surface of the receiving portion at an opening portion of the receiving portion is 2.5 µm or less.

12. The molding method for a resin molded product according to claim 6, wherein
a depth of the recessed portion from the end-surface defining surface in the axial direction is 20 µm or more.

13. The molding method for a resin molded product according to claim 6, wherein
a depth of the recessed portion from the end-surface defining surface in the axial direction is 50 µm or more.

14. The molding method for a resin molded product according to claim 6, wherein
a depth of the recessed portion from the end-surface defining surface in the axial direction is 100 µm or more.

15. The molding method for a resin molded product according to claim 6, wherein
a shape of an opening portion of the through hole and a shape of the recessed portion in a cross section orthogonal to the first direction are similar to each other, and
in the closing of the mold, in a state where the core pin is inserted into the core-pin receiving hole, the inner wall portion of the recessed portion faces and extends along a lateral surface of the core pin.

16. The molding method for a resin molded product according to claim 6, wherein
an opening portion of the recessed portion has an elliptical shape in a cross section orthogonal to the first direction.

17. The molding method for a resin molded product according to claim 6, wherein
in the first direction, the recessed portion is formed in a columnar shape extending in a direction in which the core pin is inserted into the core-pin receiving hole.

18. A mold for a resin molded product, the mold being used for molding a resin molded product by injection molding, the resin molded product having a through hole formed therein, the through hole being open in a predetermined end surface, the mold comprising:
a cavity having a space formed therein in which resin is to be injected; and
a core inserted into the space by moving relative to the cavity in a predetermined first direction, the core defining a region, into which the resin does not flow, inside the space, wherein
the core includes a core pin extending in the first direction and defining a shape of the through hole inside the space,
the cavity includes an end-surface defining surface facing the space and defining a shape of the predetermined end surface of the resin molded product,
a core-pin receiving hole is formed in the end-surface defining surface, the core-pin receiving hole including a recessed portion and a receiving portion, the recessed portion being open to the space and being recessed in the first direction, the receiving portion being open to the recessed portion, extending in the first direction, and being configured to receive a distal end of the core pin of the core inserted into the space,
an opening width of the recessed portion is set to be larger than an opening width of the receiving portion, and
a protruding portion protruding from the predetermined end surface of the resin molded product is formed along an opening edge of the through hole by the recessed portion receiving the resin injected into the space in such a manner that the resin does not reach the receiving portion.

19. A needleless injector comprising
the resin molded product according to claim 1, the resin molded product being formed as a container in which an injection intended substance is stored, wherein
the needleless injector is configured to inject the injection intended substance into a target region without passing through an injection needle.
